# EUROPEAN PATENT SPECIFICATION

(11) **EP 3 931 124 B1**
(45) Date of publication and mention of the grant of the patent: **07.05.2025**
(21) Application number: 20763599.6
(22) Date of filing: 19.02.2020
(51) Int. Cl.: B65D 65/40, B65D 81/18, A61K 9/70, A61K 31/155, A61K 47/10, A61K 47/18, A61K 47/24, A61P 31/02, D04H 1/4258, D04H 1/435

(54) **PACKAGING FOR ANTISEPTIC WIPES AND WARMING OF PACKAGED WIPES**
VERPACKUNG FÜR ANTISEPTISCHE TÜCHER UND ERWÄRMUNG VON VERPACKTEN TÜCHERN
EMBALLAGE POUR LINGETTES ANTISEPTIQUES ET CHAUFFAGE DE LINGETTES EMBALLÉES

(30) Priority: 27.02.2019 US 201916287261
(43) Date of publication of application: 05.01.2022
(73) Proprietor: Medline Industries, Inc., Northfield, IL 60093 (US)
(72) Inventor: KEHOE, Lauren, Northfield, Illinois 60093 (US); MAALOUF, Sharbel J., Northfield, Illinois 60093 (US); PARTHUN, William, Northfield, Illinois 60093 (US); CECOLA, Alana, Northfield, Illinois 60093 (US)
(74) Representative: Greaves Brewster LLP
(86) International application number: PCT/US2020/018795
(87) International publication number: WO 2020/176305

(56) References cited:
- JP-A- 2003 081 338
- JP-U- 3 176 772
- JP-U- H0 641 588
- US-A- 6 013 363
- US-A1- 2011 005 943
- US-A1- 2011 005 943
- US-A1- 2017 188 578
- US-A1- 2017 188 578

## Description

### FIELD

The disclosure is in the field of patient care products, and in various non-exclusive embodiments is specifically directed to packaging and warming of antiseptic wipes that can be used to disinfect the skin of a surgical patient.

### BACKGROUND

It is desirable for a surgical site on a patient's skin to be substantially free of dirt and active pathogens prior to surgery. Medical workers can clean the skin of a patient or the patient can perform the cleaning. Known pre-operative cleaning processes include showering, bathing, rinsing, and wiping. It is also desirable to maintain the cleanliness of a surgical site post-operation. Surgical sites are commonly cleaned using aqueous or alcohol-based antiseptic solutions, including for example solutions that contain chlorhexidine gluconate.

Wipes including antiseptic solutions can simplify the cleaning process by eliminating the need for showering, bathing, or rinsing. When a wipe is exposed to air, evaporation of volatile liquids from the wipe can cause cooling. A patient can experience discomfort when a cold wipe is used to cleanse skin, and it is common to heat antiseptic wipes. It has been observed for many known wipes packages that the wipes will dry out and become unusable after being resident in a heater for two or three days. Some wipes have a stated heated shelf life of 40 hours, which in some cases is undesirably short.

US2017/188578 purports to describe a nonwoven wipe comprising an antiseptic solution comprising a bis-(dihydropyridinyl)-decane derivative, a first fiber having a denier of about 1.5 to about 2.0, and a second fiber having a denier of about 3.0 to 3.5.

US2011/005943 relates to a multiwipe package containing a stack of wipes in a barrier packaging film.

US6013363 relates to laminate which contains the following layers: (I) a stretched microporous resin film base layer having an opacity of at least 80%; (Ia) a heat sealable adhesive resin layer on the back side of the base layer; (II) a gas barrier resin film layer on the surface side of the base layer; and (III) an inorganic oxide thin film layer on the surface side of the gas barrier resin film layer, wherein the laminate: (i) has a water vapor permeability of at most 5 g/m2x24 hr, and (ii) has an oxygen permeability of at most 5 cc/m2x24 hrxatm.

### STATEMENT OF INVENTION

The present invention relates to a package, a method of making a package and a warming cabinet as defined in the claims.

It has been discovered that one or more wipes can be placed in a package comprising a sealed container that comprises one or more materials having a low moisture vapor transmission rate (MVTR). Therefore, wipes included in such packaging can be held at elevated temperatures for long periods of time without significant loss of volatile components. The present disclosure provides a sealed container that comprises a vapor barrier film. A vapor barrier film can generally comprise a barrier layer laminated to a support film. The barrier layer can generally comprise at least one material, *e.g.* a polyethylene terephthalate film, that has a low MVTR. The support film can generally comprise at least one material, *e.g.* a polyethylene film, that provides strength and structure to the vapor barrier film. Optionally, any two or more layers or films of a vapor barrier film can be laminated together using one or more adhesives.

A method of making a package comprises disposing a vapor barrier film around at least one wipe and sealing the vapor barrier film around the wipe to form a sealed container that contains therewithin the wipe.

A package comprising at least one wipe can be heated by generally placing the package in a storage environment having a temperature above ambient temperature. In some embodiments, at least one package can be placed on a shelf of a warming cabinet comprising a heater and at least one shelf.

Also disclosed herein but not encompassed by the wording of the claims is a method of disinfecting skin which can comprise removing a package comprising wipe from a storage environment having an above-ambient temperature, removing the wipe from the package, and applying the wipe to skin.

A wipe can generally comprise at least one cloth and a cleansing composition disposed on the cloth. The wipe comprises a nonwoven cloth comprising plural fibers having a denier in the range from 2.6 to 3.7. In some embodiments, the wipe can comprise a blended cloth including first fibers and second fibers, and the first fibers differ from the second fibers in one or more of denier, chemical composition, and length. The cleansing composition comprises chlorhexidine gluconate in an amount sufficient to reduce the number of active pathogens present on skin upon contact between the cleansing composition and the skin, and water. In some embodiments the cleansing composition can further comprise a monohydric alcohol in an amount effective to provide a defoaming effect, and a wetting
agent. In some embodiments the cleansing composition comprises no monohydric alcohol.

### DESCRIPTION OF THE FIGURES

Fig. 1 is a perspective illustration of a plurality of wipes made in accordance with an exemplary embodiment of present disclosure;
Fig. 2 is a perspective illustration of a package of wipes made in accordance with an exemplary embodiment of the present disclosure; and
Fig. 3 is a perspective illustration of an exemplary warming cabinet housing a plurality of packages of wipes made in accordance with the present disclosure.

### DETAILED DESRCRIPTION

Generally, a package can comprise a sealed container having contained therein at least one wipe, and in some cases at least two wipes. A container can generally include any one or more materials that serve as a barrier during storage of the wipe, such as at ambient temperature (25° C) or temperatures above or below ambient temperature. The materials serving as a barrier can provide one or more functions such as reducing or preventing evaporation of liquid components from a wipe, reducing or preventing contamination of a wipe, and preservation of a wipe.

A container can be formed of any one or more of flexible, semi-rigid, and rigid materials. A container can generally comprise one or more layers or films of material such as one or more selected from polymeric films, metallic films, composite films, etc. In some aspects, a sealed container comprises at least one material that permits no greater than a 0.50, 0.55, 0.60, 0.65, 0.70, 0.75, 0.80, 0.85, 0.90, 0.95, 1.00, 1.05, 1.10, 1.15, 1.20, 1.25, 1.30, 1.35, 1.40, 1.45, 1.50, 1.55, or 1.60 % reduction of moisture content of a wipe during storage of the package at a temperature 51.7 °C (125 °F) for a period of 250 hours. Reduction of moisture content can be calculated determining the difference between a weight of a package comprising at least one wipe after storage at 51.7 °C (125 °F) and a weight of the package before such storage, and then dividing the difference between the weights by the weight of the package before such storage.

Polymeric films can be extruded, co-extruded, blown, cast, non-oriented, uniaxially oriented, biaxially oriented, amorphous, crystalline, etc. as may be found suitable. Examples of useful polymeric films include any one or more of polyolefin films such as polyethylene (PE) film, ultra-low density polyethylene (ULDPE) film, very low density polyethylene (VLDPE) film, low density polyethylene (LDPE) film, linear low density polyethylene (LLDPE) film, medium density polyethylene (MDPE) film, high density polyethylene (HDPE) film, polypropylene (PP) film, oriented polypropylene (OPP) film, biaxially oriented polypropylene (BOPP) film, cast polypropylene (CPP) film, cyclic olefin copolymer (COC) films, etc., polyethylene terephthalate (PET) films such as crystalline polyethylene terephthalate (CPET) film, amorphous polyethylene terephthalate (APET) film, oriented polyethylene terephthalate (OPET) film, biaxially oriented polyethylene terephthalate (BOPET) film, etc., polyamide (PA) films such as nylon film (e.g. nylon 6, 12, 66/610, 6/12 films, etc.), oriented polyamide (OPA) film, polystyrene (PS) film, polyvinyl chloride (PVC) film, polyvinylidene chloride (PVDC) film, ethylene vinyl alcohol (EVOH) film, ethylene vinyl acetate (EVA) film, ionomer films (*e*.*g*., SURYLN^{™}), cellulosic films such as cellophane, ethyl cellulose, etc. Examples of useful metallic films include foils including one or more of metals such as any one or more of aluminum, tin, copper, etc., or alloys including useful metals. Examples of useful composite films include any one or more of metallized films such as metallized PET (MPET) films, metallized PP (MPP) films, metallized PE (MPE) films, metallized PA (MPA) films, paper coated with one or more of polymeric or metallic films, ceramic coated polyester films such as SiOₓ coated PET, PA, and PP films, AlOₓ coated PET and OPA films, etc.

Any two or more films or layers included in a container can be the same or different and can be laminated or otherwise fixed together in any order. In some cases, two or more films or layers can be adhered together by any one or more of adhesive lamination, dry bond lamination, wet bond lamination, solventless lamination, extrusion lamination, hot roll lamination, hot melt seal coating, cold sealing, extrusion coating, tie layers, melting, welding, tacking, etc. An intermediate layer such as an adhesive or tie layer can be interposed between two adjacent layers to fix the layers together. Any useful adhesive, such as solvent-based, solvent-free, and aqueous adhesives can be used. Examples of adhesives include polyurethane adhesives, acrylic adhesives, polyvinyl alcohol (PVA) adhesives, ethyl vinyl acetate (EVA) adhesives, polyester adhesives, polyolefin adhesives, latex adhesives, synthetic and natural rubber adhesives, silicate adhesives such as ammonium silicate, lithium silicate, potassium silicate, sodium silicate, tetramethylammonium silicate, etc. In some cases, a primer can be applied to a surface of a film or layer to be fixed to another film or layer. Examples of useful primers include those made from polyester, polyurethane, or vinyl polymers. Useful tie layers include ethylene-acrylic acid (EAA), ethylene-grafted-maleic anhydride (AMP), ethylene-methyl acrylate (EMA), ethylene-vinyl acetate (EVA), etc.

A wipes container can take the form of a film package and can comprise a vapor barrier film including two or more different films or layers that reduce evaporation of components of a wipe at ambient and elevated temperature. A vapor barrier film can generally have any useful water vapor transmission rate (WVTR), such as a WVTR of no greater than 1.2, 1.0, 0.9, 0.85, 0.8, 0.78, 0.75, or 0.7 g/m²/24 hrs. at 37.8 °C (100 °F) and 100 % relative humidity (measured using ASTM F1249, the specification of which is incorporated herein by reference). A vapor barrier film can generally have any useful oxygen transmission rate (O₂TR), such as an O₂TR of no greater than 0.7, 0.6, 0.5, 0.4, 0.31, 0.3, or 0.25 cc/m²/24 hrs. at 22.8 °C (73 °F) and 0 % relative humidity (measured using ASTM D3985).

The vapor barrier film can have any suitable thickness, such as a thickness ranging from 30 to 200, 40 to 175, 50 to 150, 60 to 125, 70 to 115, 80 to 100, 85 to 95, 87 to 91, or 88 to 93 µm. In some embodiments, a vapor barrier film comprises a barrier layer laminated to a support film. A barrier layer can generally have a low MVTR. The barrier layer may have a thickness ranging from 1 to 50, 5 to 40, 5 to 20, 7 to 25, 10 to 15, or 12 to 13 µm. In some aspects, a support film can generally have any thickness that provides sufficient structural support for a barrier layer. A support film can generally have any useful thickness, such as a thickness ranging from 20 to 130, 30 to 120, 40 to 110, 50 to 100, 55 to 95, 60 to 90, 65 to 85, 70 to 80, 74 to 78, or 76 to 77 µm. In some embodiments, a support film comprises a LLDPE film and a barrier layer comprises a PET film. An adhesive can optionally be interposed between a barrier layer and a support film. A useful embodiment of a vapor barrier film is a High Barrier Laminate (product code: RBA-002) available from Amcor Flexibles Europe & Americas.

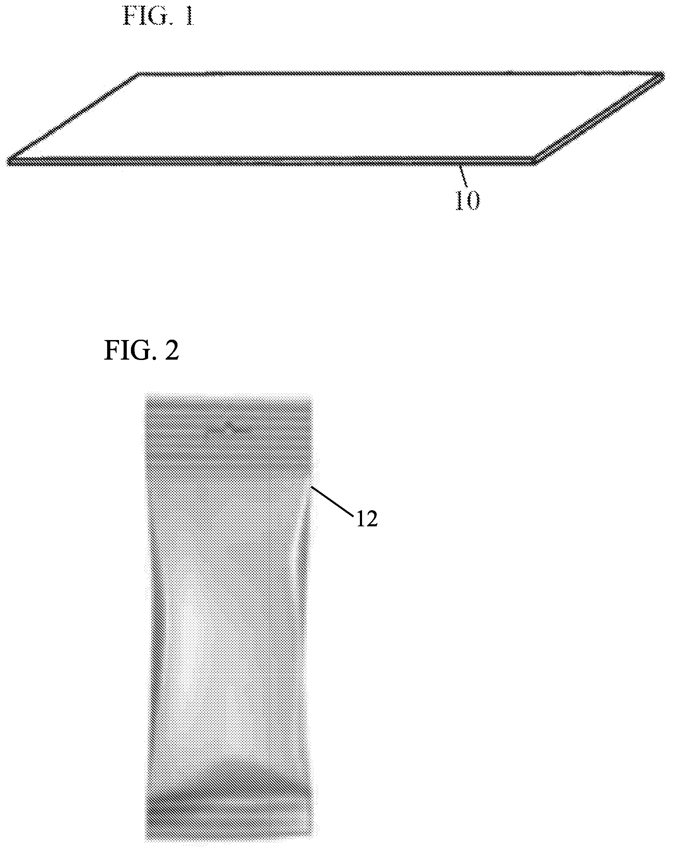

A package comprising a sealed container that contains at least one wipe can be manufactured by any useful method. Generally, a package can be made by disposing a container, or a material used to form a container, around at least one wipe and then sealing the container or the material with the wipe therein. In some embodiments, a method of making a package comprises disposing a vapor barrier film around at least one wipe and sealing the vapor barrier film to form a sealed container that contains therewithin the wipe. For example, FIG. 1 illustrates a plurality of wipes and FIG. 2 illustrates a package 12 comprising a sealed container having contained therewithin the wipes. Examples of useful methods for forming a film into a sealed container that contains at least one wipe include flow wrapping, form-fill-seal techniques such as vertical or horizontal form-fill-sealing, thermoform-fill-seal edge sealing, etc. Sealing of a film or layer to form a sealed container can generally be conducted by any useful method such as heat sealing, ultrasonic sealing, adhesive sealing, etc.

Generally, a wipe includes a cleansing composition disposed on a cloth. The cleaning composition is generally aqueous and includes purified water (USP) as a solvent or vehicle. The cleansing composition can further comprise an antiseptic, a humectant, an emollient, a wetting agent, and a monohydric alcohol as a defoamer. In some embodiments, the cleansing composition comprises no monohydric alcohol, for example, no isopropyl alcohol.

Any suitable antiseptic can be included in a cleansing composition. An exemplary antiseptic includes one or more of a biguanide, octenidine dihydrochloride, a quaternary ammonium compound, an alcohol, an iodine preparation, a peroxide, polyhexanide, a quinolone compound, an antimicrobial dye, a permanganate, a halogenated phenol compound, etc. Exemplary biguanides include chlorhexidine gluconate, chlorhexidine acetate, alexidine, derivatives and mixtures thereof. The antiseptic is included in an amount ranging from 0.01 to 10 %, 0.1 to 5 %, or 1 to 3 % by weight of the cleansing composition.

Any suitable humectant can be included in a cleansing composition to aid in increasing moisture content of skin. Exemplary humectants include any one or more of collagen, a polyhydric alcohol, urea, aloe vera, glucono-delta-lactone, etc. Useful polyhydric alcohols include butylene glycol, glycerin, hexylene glycol, polyalkylene glycols such as polyethylene glycol and polypropylene glycol, propylene glycol, dipropylene glycol, sorbitol, triethylene glycol, arylpropyne glycol, alkaline polyols 1,3-dibutylene glycol, allantoin, derivatives and mixtures thereof, etc. In some embodiments, a humectant is included in an amount ranging from 0.3 to 15 %, 1 to 10 %, or 3 to 8 % by weight of the cleansing composition. Exemplary cleansing compositions include at least one selected from glycerin and propylene glycol. An embodiment of a cleansing composition comprises both glycerin and propylene glycol.

A cleansing composition can include any suitable emollient to condition or otherwise soften skin. Exemplary emollients include any one or more of a fatty acid such as caprylic acid, lauric acid, myristic acid, oleic acid, palmitic acid, and stearic acid; a fatty ester such as glyceryl stearate, isopropyl myristate, and isopropyl palmitate; aloe vera, a fatty alcohol such as cetearyl alcohol, cetyl alcohol, lauryl alcohol, oleyl alcohol, and stearyl alcohol; a silicone such as cyclomethicone, cyclopentasiloxane, polydimethylsiloxane (dimethicone), dimethiconol, dimethicone crosspolymer, phenyl trimethicone, trisiloxane; petrolatum; derivatives thereof, etc. In some embodiments, an emollient is included in an amount ranging from 0.05 to 1 %, 0.1 to 0.8 %, 0.2 to 0.4 %, or 0.1 to 5.0 % by weight of the cleansing composition. In an exemplary embodiment, a cleansing composition includes dimethicone 350. Dimethicone 350 is available as DOW CORNING^{®} Q7-9120 Silicone Fluid, 350 cst. Another suitable dimethicone is DOW CORNING^{®} Dimethicone 365.

Any suitable wetting agent or surfactant can be included in a cleansing composition. Exemplary surfactants include any one or more of cationic surfactants such as benzalkonium chloride and cetrimonium chloride; anionic surfactants such as sodium lauryl sulfate, disodium laureth sulfosuccinate, sodium lauroyl sarcosinate, sodium cocoyl isethionate, and sodium methyl cocoyl taurate; amphoteric surfactants such as sodium lauriminodipropionate, disodium cocoamphodipropionate, and disodium lauroamphodiacetate; and non-ionic surfactants such as polysorbate 20, octoxynol-9, glyceryl oleate, and sorbitan oleate. In some embodiments, a surfactant is included in an amount ranging from 0.004 to 0.5 %, 0.02 to 0.3 %, or 0.05 to 0.15 % by weight of the cleansing composition. Exemplary cleansing compositions comprise benzalkonium chloride as a wetting agent and a secondary surfactant comprising at least one of octoxynol-9, and polysorbate 20. In an exemplary embodiment, the cleansing composition includes each of benzalkonium chloride, oxtoxynol-9, and polysorbate 20.

A cleansing composition can generally comprise chlorhexidine gluconate in an amount sufficient to reduce the number of active pathogens present on skin upon contact between the cleansing composition and the skin. In some aspects, a cleansing composition comprises or consists essentially of chlorhexidine gluconate in an amount ranging from 1 to 3%, glycerin in an amount ranging from 2 to 8%, propylene glycol in an amount ranging from 0.5 to 3%, isopropyl alcohol in an amount ranging from 0.2 to 0.8%, dimethicone in an amount ranging from 0.1% to 0.4%, benzalkonium chloride in an amount ranging from 0.02 to 0.08%, octoxynol-9 in an amount ranging from 0.005 to 0.1%, polysorbate 20 in an amount ranging from 0.005 to 0.1%, and the balance of water, all percentages being by weight of the cleansing composition.

An aqueous cleansing composition may optionally include an emulsifier in an amount effective to impart affinity of the cleansing composition to a hydrophobic cloth. Any suitable emulsifying agent may be used. Preferable emulsifying agents include fatty alcohols, such as C₁₄₋₂₂ alcohols, or alkyl aldosides, and in particular alkyl glucosides and even more particularly, C₁₂₋₂₀ alkyl glucosides. A particularly preferred emulsifying agent is cetyl alcohol. Other emulsifying agents include MONTANOV L and MONTANOV S, each available from Seppic. MONTANOV L is a proprietary mixture of C₁₄₋₂₂ alcohols and C₁₂₋₂₀ alkyl glucoside for the preparation of an O/W (oil-in-water) emulsion. MONTANOV S is a mixture of coco glucoside and coconut alcohol. The emulsifier can be present in any amount suitable for the purpose stated above. In some embodiments, the emulsifier can be present in an amount ranging from 1-2% by weight of the cleansing composition. In some cases, the emulsifier comprises at least emulsifying agents selected from among the C₁₄₋₂₂ alcohols, C₁₂₋₂₀ alkyl glucosides, coco glucosides, coconut alcohols, and mixtures thereof.

The cleansing composition may further include other ingredients such as fragrances, colors, preservatives, and additives to modify the pH. These ingredients may be added in any amounts suitable for their intended purposes.

Any suitable fragrance may be included in a cleansing composition. Exemplary fragrances include one or more selected from vanilla crème extract and chamomile extract.

One suitable preservative is EUXYL^{®} PE9010, available from Schülke & Mayr GmbH. This is a liquid cosmetic preservative that is based on phenoxyethanol and ethylhexyl glycerin. The preservative may be present in any amount effective to provide a property of preservation. For example, the preservative may be present in an amount of about 0.1% - 1.5% by weight of the cleansing composition.

The pH of a cleansing composition can, if desired, be adjusted to a range compatible with the (average) pH of healthy skin, to a range so as to compensate for the pH of residues that otherwise may remain on the skin after cleansing with a wipe, or to a range more inhospitable to pathogens. For instance, adjusting the pH to not more than 5.5 or 6 is consistent with the pH of healthy skin being approximately 5. Compensating for residues that may otherwise be alkaline can be achieved by adjusting the pH to a range less than 5, perhaps as low as 4, even down to a pH of 3 in some cases. However, too low a pH can lead to skin irritation. The pH can range from about pH 3 to about pH 9, particularly from about pH 4 to about pH 7.5. Compounds for adjusting the pH can be suitably selected from dermatologically acceptable pH control agents, agents suitable for skin care products and the like. Compounds include, for example, fruit acids such as citric acid, conjugate bases like citrates such as sodium citrate or trisodium citrate, gluconic acid, lactic acid, glycolic acid, lactates such as sodium lactates, malic acid, malates such as sodium malate, as well as mixtures of any thereof. The compound(s) to be selected should be compatible with other ingredients in a cleansing composition and may be used in any suitable amount.

The cleansing composition further may include any suitable thickener in any amount suitable to provide a thickening effect. In general, thickeners include certain ingredients that can also serve as thickeners (viscosity-increasing agents). Typically, such viscosity increasing agents include, but are not limited to, hydrogenated vegetable oils like hydrogenated jojoba oil and hydrogenated jojoba wax; microcrystalline wax; paraffin wax; beeswax; carnauba wax; ozokerite wax; ceresine wax; myristyl alcohol; behenyl alcohol; stearyl alcohol; cetearyl alcohol; hydrogels; and mixtures thereof. It will be appreciated that other modifiers that can function as thickeners may be suitably selected.

The cleansing composition can include other ingredients as suitable for their intended purposes. Other adjunctive ingredients can include texturizers, anti-oxidants, pH buffers, metal sequestrants, and anti-stick agents. These may be used in amounts consistent with their intended purposes.

As noted above, FIG. 1 depicts a plurality of wipes 10. A cloth can generally comprise plural fibers of a single type or a blend of two or more different types of fibers. A blended cloth can include two or more different types of fibers that differ in any one or more of chemical composition, denier, length, tensile strength, density, cross-sectional shape, etc. A blended cloth can generally comprise any relative amounts of the different fibers. In some embodiments, a blended cloth comprises a first type of fibers in a greater amount than a second type of fibers. Such blended cloth can generally comprise any mass ratio of two more different types of fibers, such as mass ratio of first fibers to second fibers ranging from 10:90 to 90:10, 20:80 to 80:20, 30:70 to 70:30, or 40:60 to 60:40, or being 50:50.

Suitable fibers include any one or more of hydrophobic fibers, hydrophilic fibers, natural fibers, and synthetic fibers. Examples of useful fibers included any one or more of cotton, wool, silk, jute, linen, polyester, rayon (*e.g.* lyocell), nylon, polyethene, polypropylene, polyolefin, polyamide, acrylic, acetate, spandex, latex, azlon, modacrylic, novoloid, nytril, saran, vinal, vinyon, regenerated cellulose fiber, cellulose acetate fiber, etc.

The cloth can generally include fibers having any denier. In some embodiments, a cloth comprises plural fibers having a substantially uniform denier, such that the fibers in the cloth have a denier variance ranging from 0.05 to 0.09, 0.06 to 0.08, or 0.074 to 0.078. In some embodiments a cloth comprises plural fibers having a denier ranging from 0.5 to 6.0, from 1.0 to 5.5, from 2.0 to 4.0, from 2.6 to 3.7, from 2.75 to 3.45, or from 2.8 to 3.6. In other embodiments, a cloth can comprise two or more different types of fibers having different deniers, such as a cloth comprising first fibers having a denier ranging from 0.5 to 3.0, 1 to 2.5, 1.1 to 2.0, 1.2 to 1.5, 1.2 to 1.8, or 1.3 to 1.4 and second fibers having a denier ranging from 1.2 to 7.0, 1.5 to 6.0, 2.0 to 5.5, above 3.0 to 6.0, 3.5 to 5.5, 3.8 to 5.2, 4.2 to 5.0, 4.3 to 4.9, 4.5 to 4.75, or 4.6 to 4.7. As another example, a blended cloth can comprise a first type of fibers and a second type of fibers, and the denier of the second type of fibers can range from 2 to 5 times, 2.5 to 4.5 times, or 3 to 4 times the denier of the first type of fibers.

In some embodiments, a cloth is made of plural fibers that have a substantially uniform denier and are composed of a single chemical composition, such as polyester. In some embodiments, a cloth comprises two or more different types of fibers having different deniers but the same chemical composition, such as polyester. Some embodiments of a cloth comprise two or more different types of fibers having different chemical compositions, such as polyester and rayon, but all of the fibers in the cloth have a substantially uniform denier. In yet other embodiments, a cloth comprises two or more types of fibers having different chemical compositions and different deniers.

A cloth can generally include one or more types of fibers having uniform or nonuniform lengths. In some embodiments, a cloth includes fibers having substantially uniform lengths ranging from 30 to 90 mm, 35 to 80 mm, 65 to 85 mm, 70 to 80 mm, or 74 to 76 mm. In other embodiments, a blended cloth comprises two or more types of fibers having different lengths, such as a first type of fibers having lengths ranging from 20 to 80 mm, 25 to 77 mm, 30 to 70 mm, or 35 to 65 mm, and a second type of fibers having lengths ranging from 60 to 120 mm, 70 to 110 mm, 75 to 102 mm, or 80 to 95 mm.

The cloth can be woven and made by weaving, or more preferably non-woven and formed by non-woven methods. A cloth can be flat or tufted. In some embodiments, the cloth can be formed in a sheet or mat. An exemplary nonwoven cloth is made of polyester fiber with denier ranging from 2.6 to 3.7 and fiber length from 70 to 80 mm.

Also encompassed in various alternative embodiments are a method of making a wipe, a method of warming a wipe, a warming cabinet, a method for disinfecting skin, and a method of preparing skin for surgery.

To make a wipe, generally, a cleansing composition as described hereinabove is first prepared and then disposed on the cloth. The temperature when mixing components of the cleansing composition can be any suitable temperature. The cleansing composition can be disposed on the cloth using any suitable means, such as by immersion in a bath or by spraying. A cloth as described hereinabove may be provided as a roll of material that may be cut to the desired dimensions of a wipe. A cleansing composition may be disposed on a cloth before or after cutting the cloth to the desired shape. After disposing the cleansing composition on the cloth, the prepared wipes can be stacked in a folded or non-folded orientation and packaged as consistent with the present teachings.

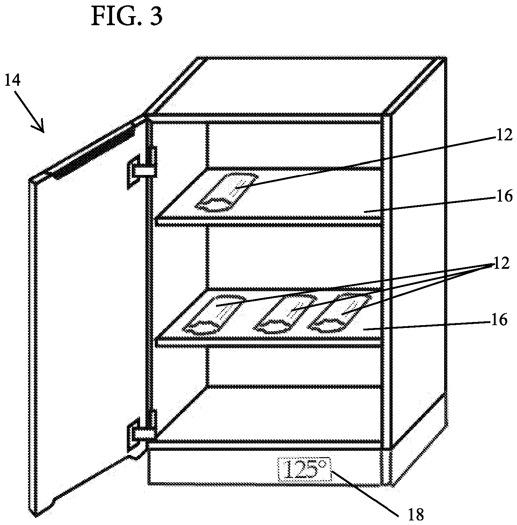

A package comprising at least one wipe can be heated by placing the package in a storage environment having any temperature above ambient temperature. Examples of useful temperatures above ambient temperature for heating packaged wipes include those ranging from 30 to 65, 35 to 60, 40 to 55, 45 to 55, or 47 to 53 °C. Generally, any type of environment can be used for heating a wipe. In some embodiments, an enclosure comprising a heater can be used to warm packaged wipes. FIG. 3 illustrates an embodiment of a warming cabinet 14 including shelves 16 and a heater with temperature gauge 18 showing a typical heated temperature of 125° C. Packages 12 comprising wipes are placed on the shelves 16.

A method of disinfecting skin can optionally include removing a package comprising at least one wipe from a storage environment having temperature above ambient temperature and removing a wipe from the package. A method of disinfecting skin can also include applying a wipe as described herein to skin. The wipe can be applied to the skin of a patient by a caretaker, a medical worker, or by the patient. When disinfecting skin, a package can be opened by any means such as tearing, cutting, rupturing, etc. the package, or opening a permanently sealed or resealable closure. After opening the package, a wipe can be removed and applied to skin. A wipe removed from a storage environment having a temperature above ambient temperature can be applied to skin before the temperature of the wipe drops to ambient temperature.

When a wipe is applied to the skin, the cleansing composition emanates from the cloth and onto the skin. The wipe described herein can be used as a pre-operative or a post-operative wipe for disinfecting skin at a surgical site. For sanitary purposes, the wipe can also be used for disinfecting skin that has not or will not undergo surgery. While wipes described herein can be applied to skin as frequently as desired, it is preferable that the wipes not be applied to a surgical site more than about eight hours prior surgery. The wipe may be applied to cleanse the skin at any suitable time before surgery, such as within one hour prior to surgery, within two hours prior to surgery, within three hours prior to surgery, within four hours prior to surgery, within five hours prior to surgery, within six hours prior to surgery, within seven hours prior to surgery, within eight hours prior to surgery, or at eight hours prior to surgery.

The following examples are provided to illustrate the present invention but should not be construed as limiting a scope of the invention.

### Example 1

A cleansing composition was made by combining 2% chlorhexidine gluconate (Relative density: 1.02, Specific gravity/density: 1.017 at 25 °C), 5% glycerin, 1.5% propylene glycol, 0.5% isopropyl alcohol, 0.75% Dimethicone 365 emulsion, benzalkonium chloride, and the balance purified water, all percentages by weight of the cleansing composition.

The obtained cleansing composition was disposed on nonwoven polyester cloth having a maximum denier of 3.45, a minimum denier of 2.75, and a target denier of 3.10, to obtain a plurality of wipes.

Two separate studies, including respectively 340 and 347 volunteer subjects, were conducted on the example wipe under randomized conditions to measure antimicrobial efficacy as specified under Tentative Final Monograph (TFM) for OTC Healthcare Antiseptic Drug Products - June 17, 1994 (TFM-1994). In both studies, the exemplary wipe was topically applied to skin by vigorously scrubbing skin in a back and forth motion for up to three minutes per treatment area (abdomen or groin). In both studies, the exemplary wipe met the required responder rates as defined under TFM-1994 for up to 8 hours.

### Example 2

Twenty-four packages were prepared. Each package included a sealed container constructed of RBA-002 high barrier laminate available from Amcor Flexibles Europe & Americas. The laminate had the following layering:

### 12 µm High Barrier PET/adhesive/76 µm White LLDPE

The laminate had a total thickness of 91 µm, a WVTR of 0.78 g/m³/24 hrs. at 37.8 °C (100 °F) and 100 % relative humidity, an O₂TR of 0.31 cc/m3/24 hrs. at 22.8 °C (73 °F) and 0 % relative humidity, and a basis weight of 94.1 g/m². Two folded wipes prepared according to Example 1 were placed in each container. The containers were then heat sealed around the wipes such that each container included one lengthwise seal and one transverse seal at each end.

The packages were weighed and then placed on separate shelves in a warming cabinet. The packages were held for 250 hours at a temperature of 51.7 °C (125 °F). The packages were then removed from the warming cabinet and re-weighed. Percentage moisture loss for each packet was calculated by determining the difference between the weight after warming and the weight before warming, and then dividing the difference in weights by the weight before warming. Moisture loss for the twenty-four packages ranged from 0.57 to 1.45 %, with an average moisture loss of 1.09% and a standard deviation of 0.28%.

Six of the packages were opened and the wipes were removed from the packages. The cleansing solutions in the removed wipes were tested for chlorohexidine gluconate content. Each of the six tested cleansing solutions included a chlorohexidine gluconate content ranging from 97.2 to 103.1 % of the target chlorohexidine gluconate content of 2 %.

### Example 3

A wipe is packaged as per Example 2, except that the wipes were formed from a blended cloth composed of different types of polymeric fibers.

### Example 4

A wipe is packaged as per Example 3, except that the cleansing composition on the wipe includes water, CHG, propylene glycol, aloe vera, dimethicone, glucono-delta-lactone, an Igepal surfactant, polysorbate 20, fragrance, and glycerin.

All percentages stated herein are weight percentages.

## Claims

1. A package comprising a sealed container having contained therewithin at least one wipe;
the wipe comprising:
a nonwoven cloth comprising plural fibers having a denier in the range from 2.6 to 3.7, the nonwoven cloth having a denier variance ranging from 0.05 to 0.09, and
a cleansing composition disposed on the cloth, the cleansing composition comprising chlorhexidine gluconate in an amount sufficient to reduce the number of active pathogens present on skin upon contact between the cleansing composition and the skin, a monohydric alcohol in an amount effective to provide a defoaming effect, a wetting agent, and water; and
the container comprising a vapor barrier film permitting no greater than a 1.60 % reduction of moisture content of a wipe during storage of the package at a temperature 51.7 °C (125 °F) for a period of 250 hours.

2. A package according to claim 1, the vapor barrier film comprising a barrier layer laminated to a support film.

3. A package according to claim 2, the support film comprising a linear low-density polyethylene film and the barrier layer comprising a polyethylene terephthalate film.

4. A package according to any of claims 2-3, the support film having a thickness ranging from 65 to 85 µm and the barrier film having a thickness ranging from 5 to 20 µm.

5. A package according to any of claims 1-4, the vapor barrier film having a water vapor transmission rate of no greater than 0.9 g/m²/24 hrs. at 37.8 °C and 100 % relative humidity.

6. A package according to any of claims 1-5, the vapor barrier film having an oxygen transmission rate of no greater than 0.4 cc/m²/24 hrs. at 22.8 °C and 0 % relative humidity.

7. A package according to any of claims 1-6, the cleansing composition comprising chlorhexidine gluconate, isopropyl alcohol, and benzalkonium chloride.

8. A package according to any of claims 1-7, the cleansing composition comprising of chlorhexidine gluconate, glycerin, propylene glycol, isopropyl alcohol, dimethicone, and benzalkonium chloride.

9. A package according to any of claims 1-8, the cleansing composition comprising of chlorhexidine gluconate in an amount ranging from 1 to 3%, glycerin in an amount ranging from 2 to 8%, propylene glycol in an amount ranging from 0.5 to 3%, isopropyl alcohol in an amount ranging from 0.2 to 0.8%, dimethicone in an amount ranging from 0.1% to 0.4%, benzalkonium chloride in an amount ranging from 0.02 to 0.08%, and water.

10. A method of making a package, the method comprising:
disposing a vapor barrier film around at least one wipe;
sealing the vapor barrier film to form a sealed container having contained therewithin the wipe;
the wipe comprising:
a nonwoven cloth comprising plural fibers having a denier in the range from 2.6 to 3.7, the nonwoven cloth having a denier variance ranging from 0.05 to 0.09, and
a cleansing composition disposed on the cloth, the cleansing composition comprising chlorhexidine gluconate in an amount sufficient to reduce the number of active pathogens present on skin upon contact between the cleansing composition and the skin, a monohydric alcohol in an amount effective to provide a defoaming effect, a wetting agent, and water; and
the vapor barrier film permitting no greater than a 1.60 % reduction of moisture content of a wipe during storage of the package at a temperature 51.7 °C (125 °F) for a period of 250 hours.

11. A method according to claim 10, the vapor barrier film comprising a barrier layer laminated to a support film.

12. A method according to claim 11, the support film comprising a linear low-density polyethylene film and the barrier layer comprising a polyethylene terephthalate film.

13. A warming cabinet comprising a heater and at least one shelf and
comprising at least one package according to claim 1 disposed on the shelf.

## Patentansprüche

1. Verpackung, einen versiegelten Behälter umfassend, mit mindestens einem darin enthaltenen Tuch;
wobei das Tuch Folgendes umfasst:
einen Vliesstoff, der mehrere Fasern mit einem Denier im Bereich von 2,6 bis 3,7 umfasst, wobei der Vliesstoff eine Deniervarianz im Bereich von 0,05 bis 0,09 aufweist, und
eine Reinigungszusammensetzung, die auf dem Tuch angeordnet ist, wobei die Reinigungszusammensetzung Chlorhexidingluconat in einer Menge, die ausreicht, um die Anzahl von auf der Haut vorhandenen aktiven Krankheitserregern bei Kontakt zwischen der Reinigungszusammensetzung und der Haut zu verringern, einen einwertigen Alkohol in einer Menge, die wirksam ist, um eine entschäumende Wirkung bereitzustellen, ein Netzmittel und Wasser umfasst; und
wobei der Behälter eine Dampfsperrfolie umfasst, die während einer Lagerung der Verpackung bei einer Temperatur von 51,7 °C (125 °F) über einen Zeitraum von 250 Stunden eine Reduzierung des Feuchtigkeitsgehalts eines Tuchs um nicht mehr als 1,60 % zulässt.

2. Verpackung nach Anspruch 1, wobei die Dampfsperrfolie eine auf eine Trägerfolie laminierte Sperrschicht umfasst.

3. Verpackung nach Anspruch 2, wobei die Trägerfolie eine lineare Polyethylenfolie niedriger Dichte umfasst und die Sperrschicht eine Polyethylenterephthalatfolie umfasst.

4. Verpackung nach einem der Ansprüche 2-3, wobei die Trägerfolie eine Dicke im Bereich von 65 bis 85 µm aufweist und die Sperrfolie eine Dicke im Bereich von 5 bis 20 µm aufweist.

5. Verpackung nach einem der Ansprüche 1-4, wobei die Dampfsperrfolie eine Wasserdampfdurchlässigkeitsrate von nicht mehr als 0,9 g/m²/24 Std. bei 37,8 °C und 100 % relativer Luftfeuchtigkeit aufweist.

6. Verpackung nach einem der Ansprüche 1-5, wobei die Dampfsperrfolie eine Sauerstoffdurchlässigkeitsrate von nicht mehr als 0,4 cm³/m²/24 Std. bei 22,8 °C und 0 % relativer Luftfeuchtigkeit aufweist.

7. Verpackung nach einem der Ansprüche 1-6, wobei die Reinigungszusammensetzung Chlorhexidingluconat, Isopropylalkohol und Benzalkoniumchlorid umfasst.

8. Verpackung nach einem der Ansprüche 1-7, wobei die Reinigungszusammensetzung Chlorhexidingluconat, Glycerin, Propylenglykol, Isopropylalkohol, Dimethicon und Benzalkoniumchlorid umfasst.

9. Verpackung nach einem der Ansprüche 1-8, wobei die Reinigungszusammensetzung Chlorhexidingluconat in einer Menge im Bereich von 1 bis 3 %, Glycerin in einer Menge im Bereich von 2 bis 8 %, Propylenglykol in einer Menge im Bereich von 0,5 bis 3 %, Isopropylalkohol in einer Menge im Bereich von 0,2 bis 0,8 %, Dimethicon in einer Menge im Bereich von 0,1 % bis 0,4 %, Benzalkoniumchlorid in einer Menge im Bereich von 0,02 bis 0,08 % und Wasser umfasst.

10. Verfahren zum Herstellen einer Verpackung, wobei das Verfahren Folgendes umfasst:
Anordnen einer Dampfsperrfolie um mindestens ein Tuch;
Versiegeln der Dampfsperrfolie, um einen versiegelten Behälter zu bilden, mit dem darin enthaltenen Tuch;
wobei das Tuch Folgendes umfasst:
einen Vliesstoff, der mehrere Fasern mit einem Denier im Bereich von 2,6 bis 3,7 umfasst, wobei der Vliesstoff eine Deniervarianz im Bereich von 0,05 bis 0,09 aufweist, und
eine Reinigungszusammensetzung, die auf dem Tuch angeordnet ist, wobei die Reinigungszusammensetzung Chlorhexidingluconat in einer Menge, die ausreicht, um die Anzahl von auf der Haut vorhandenen aktiven Krankheitserregern bei Kontakt zwischen der Reinigungszusammensetzung und der Haut zu verringern, einen einwertigen Alkohol in einer Menge, die wirksam ist, um eine entschäumende Wirkung bereitzustellen, ein Netzmittel und Wasser umfasst; und
wobei die Dampfsperrfolie während einer Lagerung der Verpackung bei einer Temperatur von 51,7 °C (125 °F) über einen Zeitraum von 250 Stunden eine Reduzierung des Feuchtigkeitsgehalts eines Tuchs um nicht mehr als 1,60 % zulässt.

11. Verfahren nach Anspruch 10, wobei die Dampfsperrfolie eine auf eine Trägerfolie laminierte Sperrschicht umfasst.

12. Verfahren nach Anspruch 11, wobei die Trägerfolie eine Folie aus Linearpolyethylen niedriger Dichte umfasst und die Sperrschicht eine Polyethylenterephthalatfolie umfasst.

13. Wärmeschrank, eine Heizung und mindestens ein Regal umfassend und mindestens eine auf dem Regal angeordnete Verpackung nach Anspruch 1 umfassend.

## Revendications

1. Emballage comprenant un récipient scellé contenant au moins une lingette ;
la lingette comprenant :
un tissu non tissé comprenant plusieurs fibres présentant un denier dans la plage de 2,6 à 3,7, le tissu non tissé présentant une variance de denier allant de 0,05 à 0,09, et
une composition nettoyante disposée sur le tissu, la composition nettoyante comprenant du gluconate de chlorhexidine en une quantité suffisante pour réduire le nombre de pathogènes actifs présents sur la peau lors du contact entre la composition nettoyante et la peau, un alcool monohydrique en une quantité efficace pour fournir un effet antimousse, un agent mouillant et de l'eau ; et
le récipient comprenant un film pare-vapeur permettant une réduction ne dépassant pas 1,60 % de la teneur en humidité d'une lingette pendant le stockage de l'emballage à une température de 51,7 °C (125 °F) pendant une période de 250 heures.

2. Emballage selon la revendication 1, le film pare-vapeur comprenant une couche barrière stratifiée sur un film de support.

3. Emballage selon la revendication 2, le film de support comprenant un film de polyéthylène basse densité linéaire et la couche barrière comprenant un film de polyéthylène téréphtalate.

4. Emballage selon l'une quelconque des revendications 2 à 3, le film de support présentant une épaisseur comprise entre 65 et 85 µm et le film barrière présentant une épaisseur comprise entre 5 et 20 µm.

5. Emballage selon l'une quelconque des revendications 1 à 4, le film pare-vapeur présentant un taux de transmission de vapeur d'eau ne dépassant pas 0,9 g/m²/24 h à 37,8 °C et 100 % d'humidité relative.

6. Emballage selon l'une quelconque des revendications 1 à 5, le film pare-vapeur présentant un taux de transmission d'oxygène ne dépassant pas 0,4 cc/m²/24 h à 22,8 °C et 0 % d'humidité relative.

7. Emballage selon l'une quelconque des revendications 1 à 6, la composition nettoyante comprenant du gluconate de chlorhexidine, de l'alcool isopropylique et du chlorure de benzalkonium.

8. Emballage selon l'une quelconque des revendications 1 à 7, la composition nettoyante comprenant du gluconate de chlorhexidine, de la glycérine, du propylène glycol, de l'alcool isopropylique, du diméthicone et du chlorure de benzalkonium.

9. Emballage selon l'une quelconque des revendications 1 à 8, la composition nettoyante comprenant du gluconate de chlorhexidine en une quantité allant de 1 à 3 %, de la glycérine en une quantité allant de 2 à 8 %, du propylène glycol en une quantité allant de 0,5 à 3 %, de l'alcool isopropylique en une quantité allant de 0,2 à 0,8 %, du diméthicone en une quantité allant de 0,1 % à 0,4 %, du chlorure de benzalkonium en une quantité allant de 0,02 à 0,08 %, et de l'eau.

10. Procédé de fabrication d'un emballage, le procédé comprenant :
la disposition d'un film pare-vapeur autour d'au moins une lingette ;
le scellement du film pare-vapeur pour former un récipient scellé contenant la lingette ;
la lingette comprenant :
un tissu non tissé comprenant plusieurs fibres présentant un denier dans la plage de 2,6 à 3,7, le tissu non tissé présentant une variance de denier allant de 0,05 à 0,09, et
une composition nettoyante disposée sur le tissu, la composition nettoyante comprenant du gluconate de chlorhexidine en une quantité suffisante pour réduire le nombre de pathogènes actifs présents sur la peau lors du contact entre la composition nettoyante et la peau, un alcool monohydrique en une quantité efficace pour fournir un effet antimousse, un agent mouillant et de l'eau ; et
le film pare-vapeur permettant une réduction ne dépassant pas 1,60 % de la teneur en humidité d'une lingette pendant le stockage de l'emballage à une température de 51,7 °C (125 °F) pendant une période de 250 heures.

11. Procédé selon la revendication 10, le film pare-vapeur comprenant une couche barrière stratifiée sur un film de support.

12. Procédé selon la revendication 11, le film de support comprenant un film de polyéthylène basse densité linéaire et la couche barrière comprenant un film de polyéthylène téréphtalate.

13. Armoire chauffante comprenant un élément chauffant et au moins une étagère et comprenant au moins un emballage selon la revendication 1 disposé sur l'étagère.
